# EUROPEAN PATENT SPECIFICATION

(11) **EP 0 866 075 B1**
(45) Date of publication and mention of the grant of the patent: **27.10.2004**
(21) Application number: 98101704.9
(22) Date of filing: 02.02.1998
(51) Int. Cl.: C07K 7/06, C07K 7/08, C07K 5/10, A61K 38/00, C12Q 1/02, C12Q 1/48, C12R 1/19

(54) **Method to treat microbial infections by uncoupling of phosphotransferase system and appropriate agents therefor**
Verfahren zur Behandlung von mikrobiellen Infektionen mittles Entkupplung des Phosphotransferase-Systems und dazu geeingnete Wirkstoffe
Procédé pour le traitement d'infections microbiennes à travers le découplage du système phosphotransphérasique et agents appropriés

(30) Priority: 19.02.1997 EP 97102616
(43) Date of publication of application: 23.09.1998
(73) Proprietor: Arpida AG,, 4142 Münchenstein (CH)
(72) Inventor: Erni, Bernhard, 3052 Zollikofen (CH)
(74) Representative: Best, Michael, Dr.

(56) References cited:
- EP-A- 0 664 450
- MAERSH P.D. ET AL.: "Inhibition by the Antimicrobial Agent Chlorhexidine of Acid Production and Sugar Transport in Oral Streptococcal Bacteria" ARCH.ORAL.MICROBIOL., vol. 28, no. 3, 1983, pages 233-40, XP000885546

## Description

The invention concerns a drug assay for the identification of novel and highly specific antimicrobials.

Infectious diseases affect all age groups and cause one-third of all death worldwide. Respiratory infections, diarrheal diseases and tuberculosis, neonatal tetanus and whooping cough are the most prominent among the bacterial infections. Infectious diseases are still rising due to population growth, urbanization, and not the least due to the widespread use of antibiotics that led to the development of antibiotic resistance. The increasing incidence of bacterial drug-resistance requires that new targets of antimicrobial therapy be identified and that inhibitors of these systems be discovered. It was found that the bacterial phosphotransferase system (PTS) is one such target. (compare Fig. 1) for the identification of novel and specific antimicrobials.

European Patent Application 0 664 450 A1 proposes to use PTS-activity of intact bacteria as a readout (a measurable parameter of an organism) to monitor the presence of cadmium, chromium mercury, copper, nickel, lead and other heavy metals as well as of water-insoluble organic solvents at toxicologically relevant concentrations in samples from the environment. It is nowhere implied that these compounds specifically inhibit any of the protein subunits of the PTS, nor that they could be used as antimicrobials. The PTS is not the target of these compounds but part of a reporter system (the other part is the 6-phospho-beta-galactosidase) to monitor the reaction of living bacteria under the assault of non-specifically toxic compounds.

In Arch. Oral. Microbiol. Vol. 28, no. 3, 1983, pages 233 to 240, Marsh *et al* demonstrate that chlorhexidine at concentrations of 0.05 mM to 0.29 mM inhibits glucose PTS activity of toluene-permeabilized oral streptococci. They report (but do not show results) that it also inhibits sugar transport by whole cells. They further show, that addition of chlorhexidine results in quick stop of acid production. Since acids are the metabolic endproducts of glucose fermentation, and since fermentation is contingent on prior uptake of glucose, cessation of acid production is taken as a readout for inhibition of PTS-mediated glucose uptake. However, cessation of acid production could also be caused by inhibition of one or several glycolytic enzymes or leakage of glycolytic intermediates across a chlorhexidine-damaged membrane.

The claim by Marsh *et al*. (1983) that chlorhexidine inhibits the PTS is not conclusive based on their own experimental data and is disproved by more recent literature. If the PTS is inhibited at all by this compound it is by the indirect effect of general cell poisoning.The effect upon the PTS is non-specific and comparable to effects induced by other membrane active cell poisons, such as uncouplers of the membrane potential. Such more recent published literature references are:
Iwami Y, Schachtele CF, Yamada T. (1995) Mechanism of inhibition of glycolysis in Streptococcus mutans NCIB 11723 by chlorhexidine. Oral Microbiol Immunol 10:360-4.
Kuyyakanond T, Quesnel LB. (1992) The mechanism of action of chlorhexidine. FEMS Microbiol Lett 79:211-5.
Fitzgerald KA, Davies A, Russell AD. (1992) Mechanism of action of chlorhexidine diacetat and phenoxyethanol singly and in combination against gram-negative bacteria. Microbios 70:215-30.

The present invention relates to a drug assay for the identification of peptides which are specific antimicrobials comprising uncoupling or inhibition of the bacterial phosphotransferase system by either
a) using such peptides as a phosphorylgroup acceptor which is not part of the system and preferably hydrolyses spontaneously or under the influence of non-specific endogenous phosphohydrolases or
b) inhibiting the phosphotransferase system activity with such peptides which block or inactivate at least one of the enzymes of the phosphotransferase system.

Furthermore, the invention relates to novel antimicrobials identified by using said drug assay. 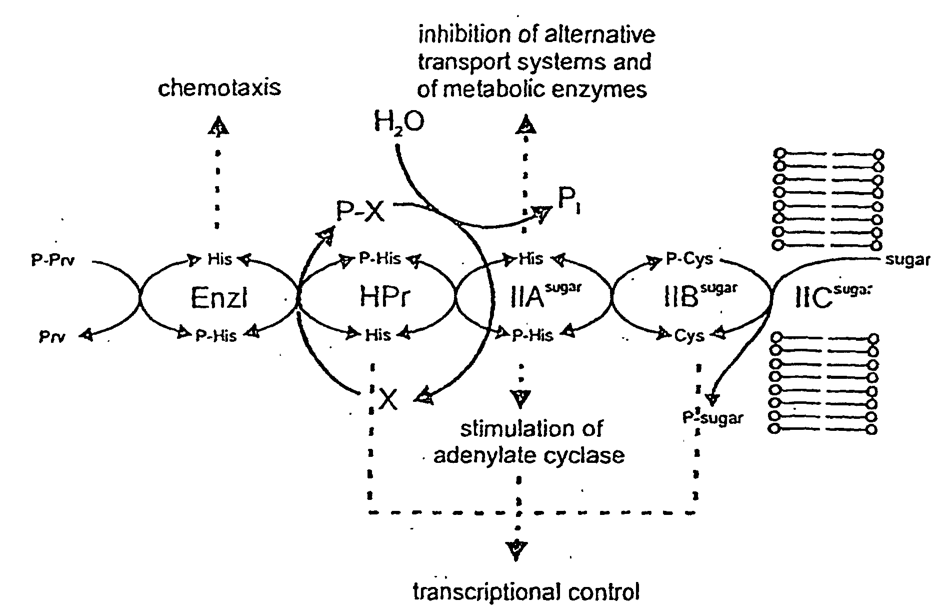 Fig. 1. The bacterial phosphoenolpyruvate dependent phosphotransferase system. Phosphorylgroups are sequentially transferred from phosphoenolpyruvate (P-Prv) to HPr and hence to the different transporters for carbohydrates and polyalcohols (IIA^{sugar}, IIB & IIC. Enzyme I, the IIA^{crr} subunit of the glucose transporter, the IIB subunit of the β-glucoside transporter of *E. coli* and of possibly other gram negative bacteria, and HPr of *B*. *subtilis* and other gram positive bacteria have regulatory functions. X is the hypothetical inhibitor. Ideally, X should be phosphorylated by enzyme I and then hydrolyse spontaneously, thereby uncoupling the protein phosphorylation.

The PTS plays a dual role in transport and phosphorylation of carbohydrates and hexitols on one hand and in signal transduction on the other. The molecular basis of its dual function is a protein phosphorylation cascade comprising four phosphoprotein units which sequentially transfer phosphoryl groups from phosphoenolpyruvate to the transported carbohydrates or polyalcohols (Fig. 1). Phosphoryltransfer occurs through phosphohistidine and phosphocysteine intermediates (for review see Meadow et al., 1990; Erni, 1992; Postma et al., 1993; Saier and Reizer, 1994: Saier et al., 1995). The first unit in the cascade phosphoenolpyruvate-protein-phosphatase termed enzyme I (Enz 1) (EC 2.7.3.9.), is a soluble two-domain protein (LiCalsi et al., 1991; Seok et al., 1996). It catalyses the phosphoryltransfer from phosphoenolpyruvate to the second component, the small (9 kD) soluble phosphorylcarrier protein HPr. HPr itself then serves as phosphoryldonor to the different PTS transporters (sugar and / or polyalcohol specific IIA IIB, IIC subunits, called phosphotransferase enzyme IIA, phosphotransferase enzyme II BC, all numbered EC 2.7.1.69; Lengeler et al., 1994). The transporters mediate Sugar and polyalcohol uptake by a mechanism that couples translocation with 5 phosphorylation of the transported solute. (Fig. 1).

In addition to the transport and the phosphorylation of sugars and / or polyalcohols the PTS also regulates a variety of physiological processes in both gram-negative and gram-positive bacteria, e. g . catabolite repression, transport of carbohydrates which are not substrates of the PTS, carbon and energy metabolism, carbon storage, the coordination of nitrogen with carbon metabolism, and chemotaxis (Powell, et al., 1995; Saier and Reizer, 1994). Signal transduction by the PTS is based on the cyclic interconversion of the proteins between phosphorylated and non-phosphorylated forms. The two forms regulate allosterically and by phosphorylation the activity of metabolic enzymes and transcription factors (for review see Postma et al., 1993). The concentration of the dephosphorylated form of PTS proteins increases when PTS sugars are present and taken up by the cell. The phosphorylated species predominate in the absence of PTS substrates. Phosphorylated IIA of gram-negative bacteria stimulates adenylate cyclase and thereby the cAMP dependent transcription of the catabolite controlled genes (Fig. 1). Dephosphorylated IIA inhibits glycerolkinase (Hurley et al., 1993), the transporters for maltose, lactose, melibiose, raffinose, and most likely other as yet unknown enzymes (for reviews see Saier, 1993; Titgemeyer et al., 1994; Saier et al., 1995).
In gram-positive bacteria HPr instead of IIA is involved in catabolite repression (Saier et al., 1995). HPr of gram-positive bacteria is phosphorylated at Ser46 by an ATP dependent HPr specific protein kinase. Phosphorylated HPr acts as corepressor by binding to the Catabolite control protein A (CcpA) repressor thereby increasing the a ffi n i ty of CcpA for the regulatory DNA region (reviewed in Saier et al., 1995).

Recent studies have uncovered that catabolite repression plays a role in the expression of virulence factors. *S. typhimurium* lacking adenylate cyclase are avirulent for mice (Curtiss and Kelly, 1987; Curtiss et al., 1988). Similarly a *S. choleraesuis* mutant lacking a functional adenylate cyclase was avirulent indicating that genes involved in cell attachment or invasion may be under catabolite control (Kelly et al., 1992). Vero cytotoxin-producing *E. coli* showed marked inhibition of adhesion when grown in the presence of the PTS sugar mannose, further suggesting that the expression of virulence genes might be controlled by the PTS via catabolite repression (Nishikawa et al., 1995).

However, it must be mentioned that the PTS functions are not necessary for growth of *E. coli* on a complex medium in the laboratory. But PTS Enz I mutants cannot grow an defined media containing PTS substrates as the only carbon source. They also do not grow or grow with difficulty on other defined media containing non-PTS substrates. As stated above, the bacterial phosphotransferase system is a target of antimicrobial therapy, in particular, since the PTS is ubiquitous in bacteria but does not occur in eukaryotic cells. This minimizes the toxic side effects. The PTS, unlike cell wall synthesis or DNA replication, continues to be active and therefore vulnerable in non growing and slow-growing cells. Metabolic energy is required in stationary phase cells long alter replication and cell division are arrested.

An antimicrobial agent targeting the PTS should have the following functional properties: (i) It should interrupt the flow of phosphorylgroups along the phosphorylation cascade with the effect that PTS proteins remain dephosphorylated. Dephosphorylated proteins erroneously signal that PTS sugars are abundant even if they are not present in the medium. As a consequence virulence factors under catabolite control cannot be expressed and alternative nutrients that might be present in the host cannot be metabolized efficiently. (ii) It should act as protein histidine phosphatase towards enzyme I or any other PTS protein. This way, it not only block but also uncouples the phosphoryl transfer chain with the effect that phosphoenolpyruvate would be consumed-in a futile cycle. This will deprive the cell of an energy carrier for substrate level phosphorylation and of an important building block for biosynthetic reactions (anaplerotic synthesis of oxalacetate, gluconeogenesis, aromatic amino acid biosynthesis). The two effects, depletion of phosphoenolpyruvate and repression of catabolite controlled genes are enough to compromise cell growth and infectivity.

Of all the PTS proteins, enzyme I is preferably - but not exclusively - the target of choice for the following reasons:
- Enzyme I has been found in all bacteria containing a PTS system. There are at least seventeen enzyme I sequences listed in the EMBL protein data bank. A comparison of the complete genomes of e. g. *Mycoplasma genitalium, Haemophilus influnezae* and the almost complete genome of E. *coli* confirms, that enzyme I and HPr are always present. For comparison, the number and types of sugar specific PTS transporters strongly varies between different species.
- Enzyme I of the PTS appears to be one of the best conserved proteins with maximal amino acid sequence similarity in the prokaryotic kingdom and minimal aminoacid sequence similarity to any protein from the eukaryotic kingdom. For this reason it can be expected, that an inhibitor / uncoupler of Enzyme I should have a strong selectivity for its target and, hence, a very reduced - if at all - side effect profile regarding eukaryotic cells.
- Enzyme I transfers phosphorylgroups from phosphoenolpyruvate to the phosphorylcarrier protein HPr. This is the first reaction in the divergent cascade (compare Fig. 1). Therefore, its inhibition has the most pleiotropic effect.

Using the above explained drug target system antibacterial compounds directed against the bacterial phosphotransferase system, preferentially, but not exclusively directed against Enzyme I have been identified. To achieve this all the relevant PTS proteins of E. *coli* shown in Fig. 1 have been purified and in vitro reconstituted. With these proteins at hand the following objectives have been achieved:
- Development of in vitro assays for the rapid screening of peptides (hereinafter also called chemical compound or compound) and peptide libraries for binding to PTS proteins, inhibition of PTS activity, and for protein phosphatase activity.
- Search for chemical compounds and peptides inhibiting the phosphoryltransfer between enzyme I and HPr (compare Table I on page 16, in which examples of such peptides are listed).
- Search for peptides with protein phosphatase activity.
- Conversion of peptides (which act as strong competitive inhibitors of enzyme I) into suicide-inhibitors and uncouplers. The construction of uncouplers involves the Substitution of the nucleophilic imidazole ring of the active histidine with an N-alkylimidazole, a thiazoliumring and / or other heterocyclic compounds of similar reactivity. Such groups react with the enzyme I bound phosphorylgroup like imidazole. However, unlike imidazolium
- phosphate, the N-alkylimidazolium/thiazolium phosphates cannot be stabilized by deprotonation and are hydrolyzed immediately. The construction of suicide inhibitors involves suitably substituted a, β- unsaturated thiols and other nucleophilic groups, which upon phosphorylation become good leaving groups (e. g. phosphate thioesters). Such leaving groups accelerate vinylogous nucleophilic substitutions by protein nucleophiles at the β-carbon.
- Search for peptides or peptide conjugates inhibiting the PTS in bacteria using a bacterial screening assay for the discovery of chemical compounds which specifically inhibit the PTS.

The following exemplification illustrates the invention

Assays for the screening of chemical compound libraries for binding to PTS proteins, inhibition of PTS activity, and for protein phosphatase activity.

Several assays (Fig. 2 on the next page) were developed to identify chemical compounds which (a) bind to Enzyme I and (b) are phosphorylated by Enzyme I. A further assay (c) to detect ligands with phosphatase activity towards Enzyme I has also been developed (for details see *Materials and Methods).*

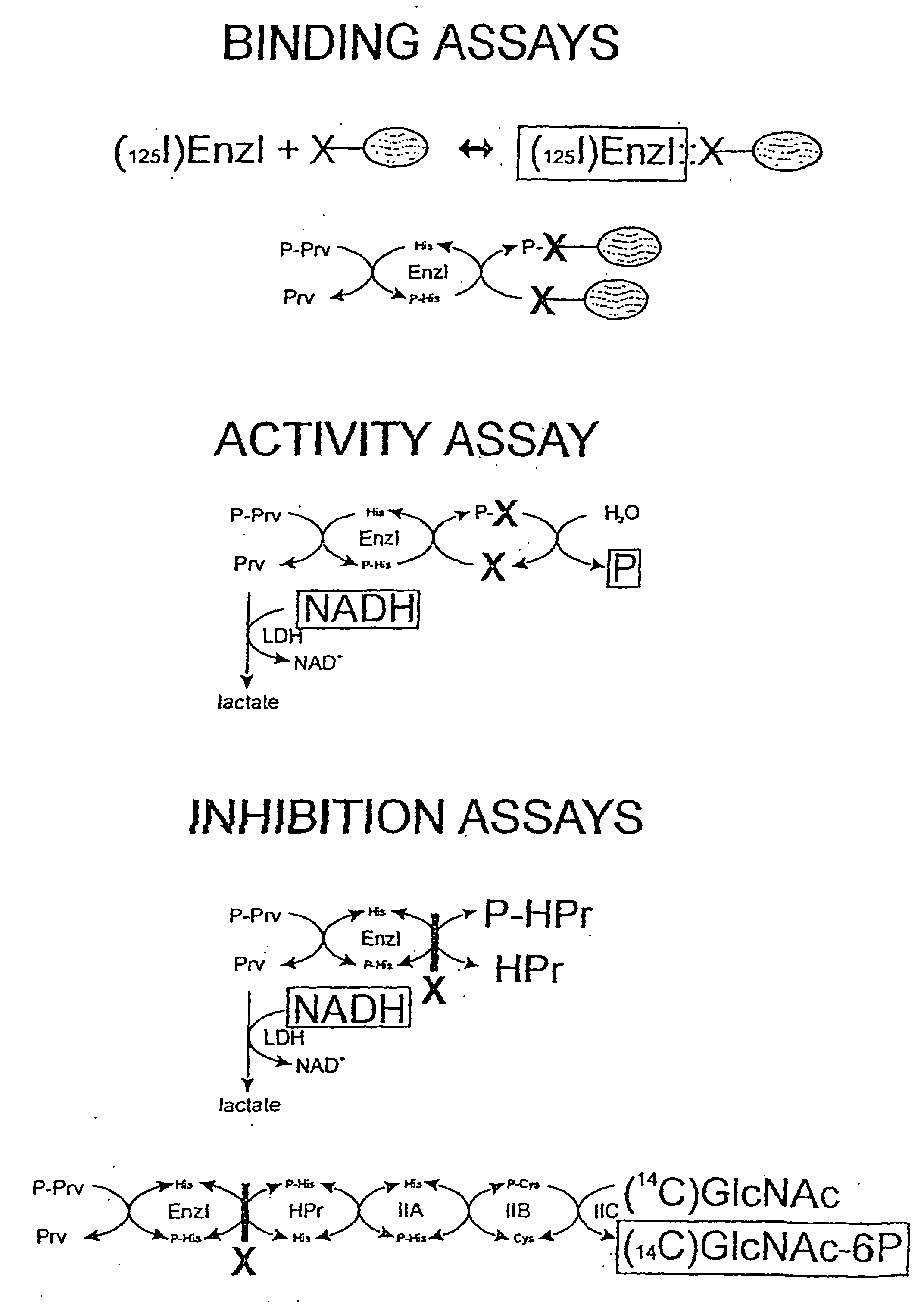

Fig. 2. Assays for the screening, selection and characterization of inhibitory compounds from combinatorial libraries. Reaction products that can be determined experimentally are boxed. The inhibitory compound X can be in soluble form, immobilized on a filamentous phage, or bound to a microtiter well. The transporter for GlcNAc (shaded in grey) contains the IIA, IIB and IIC functional units as domains of a single polypeptide chain.
(*a*) *Binding of Enzyme I* to immobilized ligands, e.g. filamentous phages from a phage display library or synthetic peptides from a combinatorial peptide library was detected using [¹²⁵1]-Enzyme I and autoradiography.
(b) *Phosphorylation by Enzyme I*. To identify peptides and filamentous phages displaying a peptide which can be phosphorylated by Enzyme 1, support based peptide libraries and immobilized peptide-displaying phages were incubated over night with enzyme I in the presence of [³²P]phosphoenolpyruvate, and the phosphorylated species were detected with a PhosphorImager. The enzyme I dependent phosphorylation was confirmed by thin layer chromatography, autoradiography and by electrospray mass spectroscopy of the phosphorylated soluble peptides.
c) *Phosphatase activity*. A conventional and well established assay to colorimetrically detect the formation of inorganic phosphate has been adapted to the specific needs. The assay can be performed in microtiter plates and quantitated with a microplate photoreader. Another assay to monitor phosphatase activity of soluble compounds is based on the detection of pyruvate which is continuously produced from phosphoenolpyruvate whenever enzyme I is dephosphorylated. The enzyme I-specific phosphatase activity of soluble compounds can be detected from the pyruvate burst. The rate of pyruvate formation is monitored with lactate dehydrogenase and NADH. The assay can be performed in microtiter plates and quantitated with a microplate photoreader.
(*d*) *Inhibition of in vitro PTS activity*. The inhibitory activity of soluble compounds can be measured quantitatively in the sugar phosphotransferase assay in the presence of the purified PTS proteins and with [¹⁴C]GlcNAc ([¹⁴C]N-acetyl-glucosamine) or (14C] Glc ([¹⁴C]glucose) as terminal phosphate acceptor (Emi et al., 1982).
(*e*) *Inhibition of PTS activity in bacteria*. Compounds that specifically interfere with the PTS can also be detected *in bacteria*. Indicator E. *coli* (or any other bacteria) will be plated an two defined minimal salts media containing either glucose or a nonPTS Substrate e.g. lactose as the only carbon source. Compounds from chemical libraries are applied to both cultures. Agents that inhibit growth an glucose only but do not *affect* growth an non-PTS substrates are candidate PTS inhibitors.

### Peptides from phage display libraries

M13 phage display libraries expressing 6-mer, 9-mer, 10-mer and 15-mer inserts (Scott and Smith, 1990; Christian et al., 1992) were panned with enzyme I to enrich for enzyme I binding phages. *E. coli* clones transfected with "high affinity" phages were grown on nitrocellulose sheets, and the secreted and nitrocellulose bound phages were incubated over night with enzyme I in the presence of [³²P]phosphoenolpyruvate. Phosphorylated phages were identified with the PhosphorImager (commercially available from e. g. Molecular Dynamics) and sequenced. The sequences of the peptides which were phosphorylated 25 to 30 fold over the average are shown in Table I. Fig. 3 on the next page illustrates this procedure.

All the phage displayed peptides contain either a histidine or a cysteine residue and all the peptides contain one or several basic residues. This is indicative of electrostatic stabilisation of the enzyme I-ligand complex between the positively charged peptide and the active site pocket of enzyme I which contains a high proportion of acidic residues (Seok et al., 1996). It is of particular interest that cysteine containing sequences appeared in addition to the expected His containing peptides. Indeed, certain PTS proteins (IIB domains of the PTS transporters, Fig. 1) are phosphorylated at cysteine (Meins et al. 1991, Eberstadt et al. 1996). It appears that the active site of enzyme I recognizes not only His but also Cys in an appropriate sequence context.

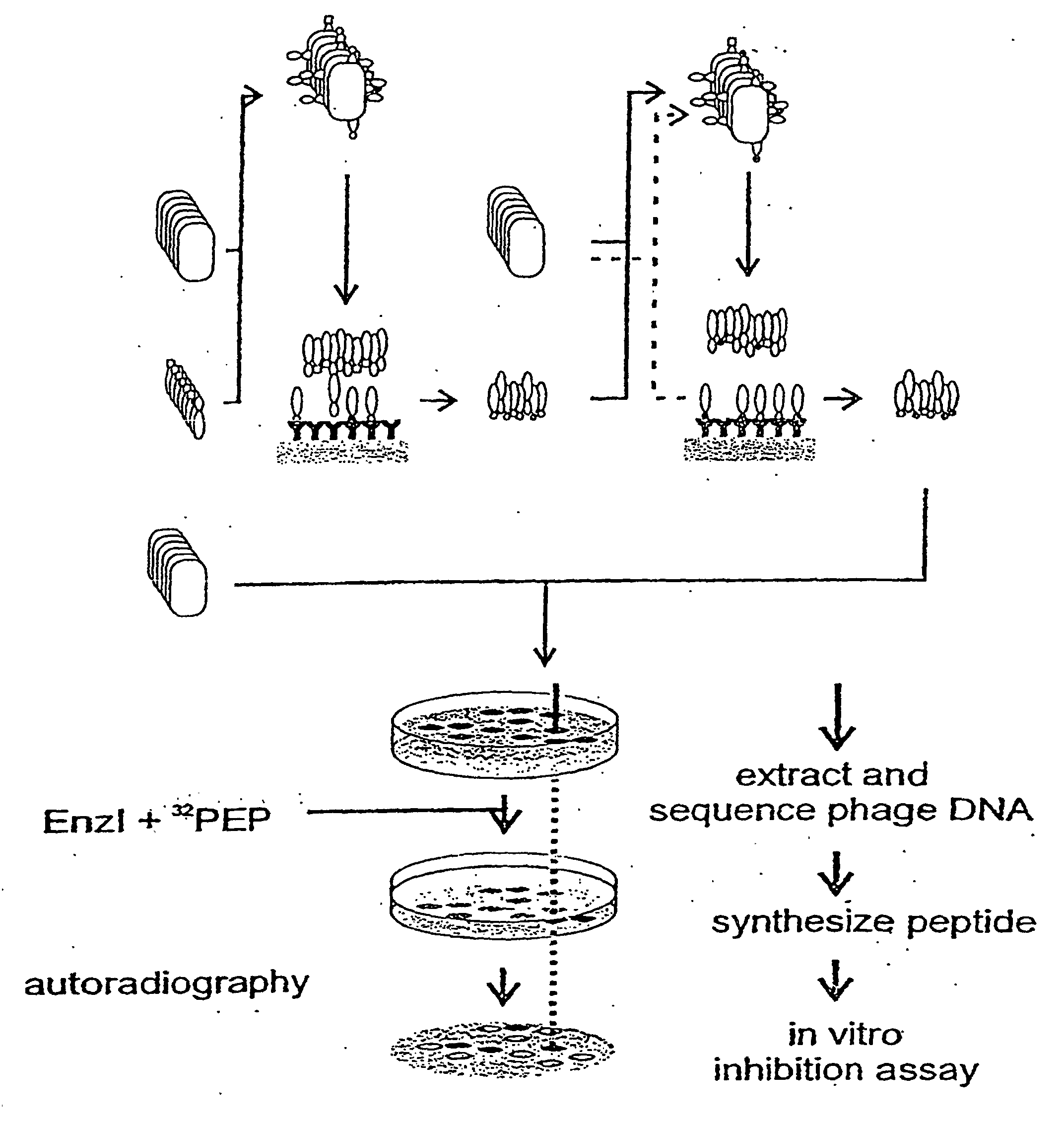

Fig. 3. Selection of Enzyme I-binding peptides from phage display libraries. M13 phages were panned with enzyme I (top of figure) to enrich for enzyme I binding phages. *E*. *coli* clones transfected with "high affinity" phages were then grown on nitrocellulose sheets, and the secreted and nitrocellulose bound phages were incubated over night with an overlay of enzyme I in the presence of [³²P]phosphoenolpyruvate. Phosphorylated phage were identified with the PhosphorImager and sequenced. The phage displayed sequences which became phosphorylated 25 to 30 fold over average were chemically synthesized and their biological activity was characterized. Results are summarized in Table 1.

### Inhibition of the PTS by peptides

Some of the peptides from the phage display library which became phosphorylated by Enzyme I have been synthesized by standard methods in soluble form and characterized. They inhibit the *in vitro* phosphotransferase activity with 1C50 of between 60 µM and over 10 mM (Table 1). The second part of Fig. 3 an the previous page illustrates this procedure.

### MATERIALS AND METHODS

*Binding of enzyme I to immobilized peptides, monitored with [*^{*125*}*I]-enzymel*. Enzyme I was radiolabeled with ¹²⁵I by the lactoperoxidase-catalyzed reaction following the procedure reported for gonadotropins (Sairam, M.R. 1979). Briefly, 1 mg of Enzyme I in 200 µl of buffer was iodinated with 10 µl (0.5 mCi) of Na ¹²⁵I. The reaction was conducted at 34°C for 60 min in a water bath with gentle shaking throughout. The reaction was terminated by the addition of potassium iodide. Iodinated protein was separated from free iodine on a Sephadex G-50 column presaturated with BSA and equilibrated with 50 mM PBS (pH 7.2).

Cellulose membranes with immobilized peptides were incubated with 50 mM TBS containing 3% BSA for 1 hr with shaking at room temperature. Membranes were subsequently washed and further incubated for 1 hr with 50 mM PBS containing 0.15% casein, another blocking agent to block the non-specific binding sites. These membranes were then incubated with [¹²⁵I]-enzyme I in PBS containing 0.15% casein for 2 hr. Non-specifically and weakly bound [¹²⁵I]-enzyme I was removed from the membranes by washing them with PBS containing 0.1% Tween. Radioactivity was determined by autoradiography. *Enzyme I dependent phosphorylation of immobilized peptides* [³²P]- PEP was prepared as described by Roossien *et al*., 1983. Cellulose membranes with immobilized peptide libraries were treated with the blocking agents as described above. These libraries were then incubated for 30 min at room temperature with [³²P]- PEP in the presence of catalytic amounts of enzyme I. The phosphorylated species were identified with a PhosphoImager ( Molecular Dynamics).

*Inhibition of phosphotransferase activity by soluble peptides*. This assay measures the inhibition of enzyme I dependent sugar phosphorylation activity. The IC₅₀ is defined as the concentration of the inhibitor required for 50% inhibition of the PTS activity. The purified N-acetylglucosamine transporter (II^{GlcNAc}) of *E*.*coli* (Mukhija and Erni, 1996) was used as the sugar specific component. The reaction mixture contained per 100 µl, 100 µg phosphatidyl glycerol from egg yolk (Sigma), 50 mM KPi, pH 7.5, 2.5 mM dithiothreitol, 2.5 mM NaF, 5 mM MgCl₂, mM phosphoenolpyruvate (potassium salt, Sigma), 0.5 mM [U-¹⁴C]GlcNAc (New England Nuclear; 56.3 mCi/mmol, diluted to 1000 cpm/nmol), and 3 µM each of purified HPr and enzyme I (Mao *et al*., 1995). The reaction was started by addition of 0.1 µg of II^{GlcNAc}. After incubation for 30 min at 37°C, the reaction was stopped by adding 1 ml ice-cold water. GlcNAc-6-Phosphate was separated from free GlcNAc by anion-exchange chromatography on AG-1-X2 (50-100 mesh, Biorad). Activity is expressed as nmoles of sugar-P formed after 30 min. The HPr concentration was adjusted such that it was rate limiting in the overall phosphotransferase reaction. The inhibitory peptides were added to concentrations of 25 µM to 10 mM. Phosphorylation of GlcNAc in the absences of peptide was taken as 100%.

*Pyruvate formation coupled to lactate dehydrogenase*. Pyruvate formed from phosphoenolpyruvate by enzyme I was converted to lactate in the presence of lactate dehydrogenase, and the concomittant consumption of NADH was followed spectrophotometrically at 340 nm. 1 ml of the reaction mixture contained 0.8 mM PEP, 0.2 mM NADH, 30 µg lactate dehydrogenase, 50 mM KPi pH 7.5, 2.5 mM dithiothreitol, 2.5 mM NaF, 5 mM MgCl₂, and as substrate either 22.5 µM HPr or 50 to 150 µM of the peptide. The reaction was started by the addition of 0.75 µM enzyme I. To measure the inhibition of enzyme I by the peptides, peptides were added at different concentrations. The inhibitory peptides are phosphorylated by enzyme I at a much slower rate than HPr.

*Growth inhibition assay*. An over night culture of *E. coli* K-12 UT580 grown in LB-medium was diluted 1:30 into soft agar (0.7% agarose in LB broth) at 40°C, and 3 ml of the diluted cell suspension were spread an LB agar plates. Filter discs (4.4 mm diameter) were placed in regular intervals on the solidified top agar. To each filter was added 10 µl of one of the different peptide stock solutions (20 µM to 80 µM). The agar plates were incubated over night at 37°C. The area of the clear zone around each filter disc was determined. The clear area is taken as a measure for the growth inhibitory activity of the peptide. Compare Fig 4 and Table 1 below and on the following two pages.

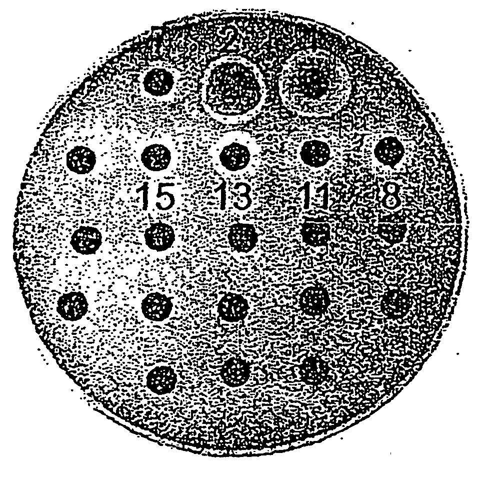

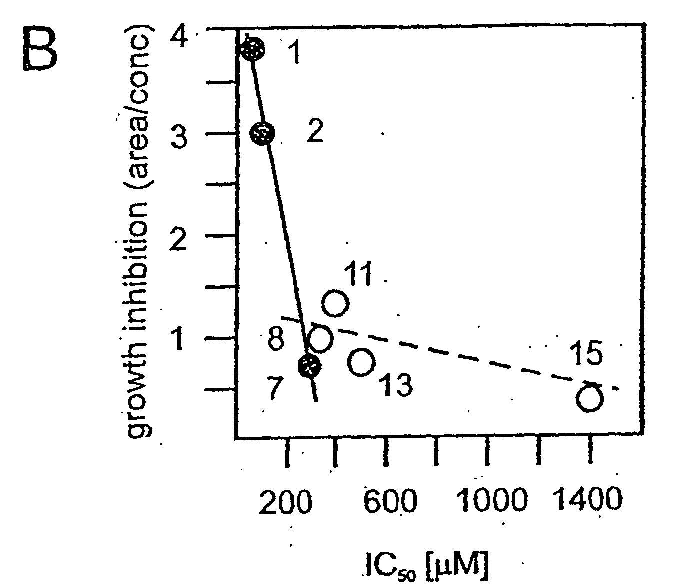

Fig. 4. Growth inhibition of E. *coli* K12 UT580. Peptides were added to filter discs and placed onto a cell culture in LB soft agar. The clear zones are indicative of growth inhibition (panel A). The growth inhibitory activity of the peptides is inversely correlated with their IC₅₀ (panel B). The numbers refer to the peptides listed in Table 1.

**Table I**

| **Biological activities of peptides identified from support-based combinatorial libraries.** | | | | |
|---|---|---|---|---|
| ID* | peptide sequence | inhibition IC₅₀ (µM) | uncoupling P;Ipeptide/h | growth inhibition MIC (µM) |
| 1 | KKFHLRK | 150 | 0.49 | 125.8 |
| 2 | KKFALRK^{a} | none | | |
| 3 | KKFDLRK^{b} | 820 | 0.05 | |
| 5 | KKWHLRKR | 30 | 0.3 | 63.0 |
| 6 | KGWHKRKK | 530 | 0.23 | |
| 7 | KKWHRRKK | 300 | 0.29 | |
| 8 | KKWHKRKK | 440 | 0.215 | |
| 9 | KKFHIRKR | 160 | 0.31 | |
| 10 | PNGLHTRPA^{d} | 2600 | 0.07 | |
| 11 | Bio-PNGLHTRPA^{e} | 4300 | - | |
| 12 | W H KR | 4900 | - | |
| 13 | Ac-WH KR^{f} | 5800 | - | |
| | HPr protein | | 2.70 | |
| | tetracycline | | | 2.5 |

| | | | | |
|---|---|---|---|---|
| ^{*a*} *peptide with no phosphorylatable amino acid residue, derived from peptide 1* | | | | |
| ^{*b*} *peptide with aspartate in place of histidine, derived from peptide 1.* | | | | |
| ^{*d*} *peptide with the sequence of HPr active site loop*. | | | | |
| ^{*e*} *biotinylated peptide to standarize in vitro enzymatic assays in micro titer* plates. | | | | |
| ^{f}Ac: acetylated peptide. | | | | |
| ***The ID number corresponds to the SEQ ID NO: outlined an pages 23-33** | | | | |

**Table II**

| **Biological activities of peptides identified by phage display** | | | | |
|---|---|---|---|---|
| **ID**** | **peptide sequence** | **inhibition IC**_{**50**} **(µM)** | **uncoupling P**_{**i**}**/peptide (x IC**_{**50**}**/h)**^{***c***} | **growth inhibition (area/µM)** |
| 14 | GLRFGKTRVHYLVLG | 25 | 3.2 | 3.65 |
| 15 | SGRKSTRVHHWLLVL | 60 | 6.6 | 3.05 |
| 16 | KMSRHRKPGA | 100 | 22.2 | |
| 17 | SSLRGHRWVY | 120 | 50.3 | |
| 18 | KISRHGKRGK | 140 | 1.9 | |
| 19 | KISRHGRPTG | 1065 | 479.3 | |
| 20 | RIHFIPRRGR | 280 | 39.6 | 0.77 |
| 21 | RLHYLF^{*a*} | 375 | 64.2 | 0.90 |
| 22 | DGARLHYLF^{*a*} | 1200 | 132.3 | |
| 23 | AcDGARLHYLF^{*a*} | 7600 | 15.2 | |
| 24 | RHWSIF | 400 | | 1.30 |
| 25 | RHRTLF | 410 | | |
| 26 | RHYLLF | 500 | | 0.83 |
| 27 | RHITSL | 650 | | |
| 28 | RHITLF | 1380 | | 0.27 |
| 29 | MSRHRN | 760 | 143.8 | |
| 30 | PNGLHTRPA^{*b*} | 2600 | | |
| 31 | MRLLKTLCFGLCG | 3500 | | |
| 32 | AcMRLLKTLCFVGLCG | >10'000 | | |

| | | | | |
|---|---|---|---|---|
| ^{*a*} Peptides Nr. 22 and 23 have at their amino terminus three extra residues (DGA) that are encoded by the coat protein pIII of M13. Ac; acetyl-. | | | | |
| ^{*b*} The amino acid sequence of peptide Nr 30 is identical with the sequence of the active site loop of HPr. Since this sequence was not found among the phage displayed peptides it was chemically synthesized and used for comparison. | | | | |
| ^{c}The rate of uncoupling is very low. It is calculated as µM NADH consumed per µM peptide per one hour multiplied by the IC₅₀.(µM). This modification allows to compare the approximate rates of phosphohydrolysis in spite of the widely different affinities (IC50) of the peptides for enzyme. | | | | |
| ****The ID number corresponds to the SEQ ID NO: outlined on pages 23-33** | | | | |

### REFERENCES

Ammer, J., Brennenstuhl, M., Schindler, P., Höltje, J.V., and Zähler, H. (1979) Phosphorylation of streptozotocin during uptake via the phosphoenolpyruvate:sugar phosphotransferase system in *Escherichia coli*. Antimicrob. Agents Chemother. 16, 801-807
Amster-Choder, O. and Wright, A. (1993) Transcriptional Regulation of the bgl Operon of *Escherichia-Coli* involves Phosphotransferase system-mediated phosphorylation of a Transcriptional Antiterminator. J. Cell. Bioch. *51*, 83-90
Buhr, A., Daniels, G.A., and Erni, B. (1992) The glucose transporter of *Escherichia coli*. mutants with impaired translocation activity that retain phosphorylation activity. J. Biol. Chem. 267, 3847-3851
Christian, R.B., Zuckermann,R.N., Kerr,J.M., Wang, L., and Malcolm, B.A. (1992) Simplified methods for construction, assessment and rapid screening of peptide libraries in bacteriophages. J. Mol. Biol. 227, 711-718.
Curtiss, R. and Kelly, S.M. (1987) *Salmonella typhimurium* deletion mutants lacking adenylate cyclase and cyclic AMP receptor protein are avirulent and immunogenic. Infect. Immun. 55,30355-3043.
Curtiss, R., Goldschmidt, R.M., Fletchall, N.B., and Kelly, S.M. (1988) A virulent *Salmonella typhimurium* delta cya delta crp oral vaccine strains expressing a streptococcal colonization and virulence antigen. Vacine 6, 155-160.
Eberstadt, M., Golic Grdadolnik, S., Gemmecker, G. Kessler, H., Buhr, A. and Erni, B (1996) Solution structure of the IIB domain of the glucose transporter of Escherichia coli. Biochemistry, in press
Eberstadt, M., Gemmecker, G. Golic Grdadolnik, S.,Kessler, H., Buhr, A., Lanz, R. and Erni, B. (1996) The glucose trasnporter of Escherichia coli: NMR characterization of the phosphocysteine form of the IIB^{Glc} domain and its binding interface with the IIA^{Glc} subunit. submitted
Erni, B. (1992) Grouptranslocation of glucose and other carbohydrates by the bacterial phosphotransferase system. Int. Rev. Cytol. 137A, 127-148
Emi, B., Trachsel, H., Postma, P.W., and Rosenbusch, J.P. (1982) Bacterial phosphotransferase system. Solubilization and purification of the glucose-specific enzyme II from membranes of *Salmonella typhimurium*. J. Biol. Chem. 257, 13726-13730
Felici, F., Luzzago, A., Folgori, A., and Cortese, R. (1993) Mimicking of discontinuous epitopes by phage-displayed peptides, II. Selection of clones recognized by a protective monoclonal antibody against the Bordetella pertussis toxin from phage peptide libraries. Gene 128, 21-27
Hurley, J.H., Faber, H.R., Worthylake, D., Meadow, N.D., Roseman, S., Pettigrew, D.W., and Remington, S.J. (1993) Structure of the regulatory romplex *of Escherichia coli* II^{Glc} with glycerol kinase. Science *259*, 673-677.
Jia, Z.C., Quail, J.W., Waygood, E.B., and Delbaere, L.T.J. (1993a). The 2.0-angstrom resolution structure *of Escherichia coli* Histidine-Containing Phosphocarrier Protein HPr - A redetermination. J. Biol. Chem. *268*, 22490-22501.
Jia, Z.C., Vandonselaar, M., Quail, J.W., and Delbaere, L.T.J. (1993b) Active-Centre torsion-angle strain revealed in 1.6 Angstrom-resolution structure of histidine containing phosphocarrier protein. Nature *361*, 94-97.
Kalbitzer, H.R. and Hengstenberg, W. (1993) The Solution Structure of the histidine containing protein (HPr) from *Staphylococcus-aureus* as determined by 2 dimensional H-1-NMR Spectroscopy. Eur. J. Biochem. *216*, 205-214.
Kelly, S.M., Bosecker, B.A. Curtiss, R. (1992) Characterization of protective properties of attenuated mutants of *Salmonella choleraesuis*. Infect. Immun. 60, 4881-4890
Lengeler, J.W., Jahreis, K. and Wehmeier, U.F. (1994) Enzymes II of the phosphoenol pyruvate-dependent phosphotransferase systems: their structure and function in carbohydrate transport. Biochem. Biophys. Acta 1188, 1-28.
Liao, D. I., Silverton, E., Seok, Y.J., Lee B. R., Peterkofsky, A., Davies. D.R. (1996) The first step in sugar transport: crystal structure of the amino terminal domain of enzyme I of the *E. coli* PEP: sugar phosphotransferase system and a model of the phosphotransfer complex with HPr. Structure, 4, 861-872.
LiCalsi, C., Crocenzi, T. S., Freire, E.and Roseman, S. (1991) Sugar transport by the bacterial phosphotransferase system. Structural and thermodynamic domains of Enzyme I of *Salmonella typhimurium*. J. Biol. Chem. 266, 19519-19527
Lux, R., Jahreis, K., Bettenbrook, K., Parkinson, J.S. and Lengeler, J.W. (1995) A communication link between the PTS carbohydrate uptake System and the chemotaxis signaling pathway of *Escherichia coli.* Proc. Natl. Acad. Sci. USA 92, 11583-11587
Mao, Q., Schunk, T., Flükiger, K., and Erni, B. (1995) Functional reconstitution of the purified mannose phosphotransferase system of *Escherichia coli* into phospholipid vesicles. J. Biol. Chem. 270, 5258-5265
Martin-Verstraete, I., Debarbouille, M., Klier, A., and Rapoport, G. (1994) Interactions of wild-type and truncated LevR of *Bacillus subtilis* with the upstream activating sequence of the levanase operon. J. Mol. Biol. 241, 178-192.
Mayahara, H., Hirano, H., Saitoh,T., and Ogawa, K. (1967) The new lead citrate method for the ultrachemical demonstration of activity of non-specific alkaline phosphatase. Histochemie 11, 88.
Meins, M., Jenö, P., Müller, D., Richter, W.J., Rosenbusch, J.P., and Erni, B. (1993) Cysteine phosphorylation of the glucose transporter of *Escherichia coli*. J. Biol. Chem. 268, 11604-11609
Meadow, N.D., Fox, D.K. and Roseman, S. (1990) The bacterial phosphoenolpyruvate:glycose phosphotransferase system. Annu. Rev. Biochem. 59, 497-542.
Mukhija, S. and Erni, B. (1996) Purification by Ni²⁺ affinity chromatography, and functional reconstitution of the transporter for N-acetylglucosamine of *Escherichia coli*. J. Biol. Chem. 271, 14819-14824
Nishikawa,Y., Scotland, S.M., Smith, H.R. Willshaw, G.A. and Rowe, B. (1995) Catabolite repression of the adhesion of Vero cytotoxin-producing *Escherichia coli* serogroups 01557 and 0111. Microb. Pathog. 18, 223-229.
Postma, P.W., Lengeler, J.W. and Jacobson, G.R. (1993) Phosphoenolpyruvate: carbohydrate phosphotransferase systems in bacteria. Microbiol. Rev. 57, 543-594.
Powell, B. S., Court, D. L., Inada, T., Nakamura, Y., Michotey, V., Cui, X., Reizer, A., Saier, M. H., and Reizer, J. (1995) Novel proteins of the phosphotransferase system encoded within the *rpoN* operon of *Escherichia coli.* Enzyme IIA^{Ntr} affects growth an organic nitrogen and the conditional lethality of the *era*^{*ts*} mutant. J. Biol. Chem. 270,4822-4839
Rajagopal, P., Waygood, E.B., and Klevit, R.E. (1994) Structural consequences of histidine phosphorylation: NMR characterization of the phosphohistidine form of histidine-containing protein from *Bacillus subtilis* and *Escherichia coli*. Biochemistry 33, 15271-15282.
Roossien, F. F., Brink, J., and Robillard, G. T. (1983) A simple method for the synthesis of ³²P phosphosphoenolpyruvate via the pyruvate kinase exchange reaction at equilibrium. Biochim. Biophys. Acta. 760,185-187
Reizer, J., Hoischen, C., Reizer, A., Pham, T.N., and Saier, M.H. (1993) Sequence Analyses and Evolutionary Relationships Among the Energy-Coupling Proteins Enzyme-I and HPr of the Bacterial Phosphoenolpyruvate-Sugar Phosphotransferase System. Protein Sci. 2, 506-521.
Saier, M.H. (1993). Regulatory Interactions Involving the Proteins of the Phosphotransferase System in Enteric Bacteria. Journal. of Cellular. Biochemistry *51*, 62-68..
Saier, M. H. and Reizer, J.(1994) The bacterial phosphotransferase system: New frontiers 30 years later. *Molecular. Microbiology*. 13, 755-764
Saier, M.H., Chavaux, S., Deutscher, J., Reizer, J., and Ye, JJ. (1995) Protein phosphorylation and regulation of carbon metabolism in Gram-negative versus Gram-positive bacteria. TIB S 20, *267*-*271*.
Scott, J.K., and Smith, G.P. (1990) Searching for peptide ligands with an epitope library. Science 249, 386-390.
Seok, Y. J., Lee, B. R., Zhu, P. P.and Peterkofsky, A.(1996) Importance of the carboxyl-terminal domain of enzyme 1 of the *Escherichia. coli* phosphoenolpyruvate: Sugar phosphotransferase System for phosphoryl donor specificity. *Proc*. *Natl*. *Acad. Sci. USA* 93,347-351
Titgemeyer, F., Mason, R.E. and Saier, M.H. (1994) Regulation of the raffinose permease of *Escherichia coli* by the glucose-specific enzyme IIA of the phosphoenolpyruvate:sugar phosphotransferase system. J. Bacteriol. 176, 543-546
van Nuland, N.A.J., Boelens, R., Scheek, R.M., and Robillard, G.T. (1995) High-resolution structure of the phosphorylated form of the histidine-containing phosphocarrier protein HPr from *Escherichia coli* determined by restrained molecular dynamics from NMR-NOE data. J. Mol. Biol. 246, 180-193.
van Nuland, N.A.J., Grotzinger, J., Dijkstra, K., Scheek, R.M., and Robillard, G.T. (1992) Determination of the 3 -dimensional solution structure of the histidine containing phosphocarrier protein HPr from *Escherichia coli* using multidimensional NMR spectroscopy. Eur. J. Bioch. *210*, 881-891.
van Nuland, N.A.J., Hangyi, I.W., van Schaik, R.C., Berendsen, H.J.C., van Gunsteren, W.F., Scheek, R.M., and Robillard, G.T. (1994) The High-Resolution Structure of the Histidine-Containing Phosphocarrier Protein HPr from *Escherichia coli* determined by restrained molecular dynamics from Nuclear Magnetic Resonance Nuclear Overhauser effect data. J. Mol. Biol
Phosphorylation of a Transcriptional Antiterminator. J. Cell. Bioch. *51*, 83-90.
Weber, L., Wallbaum, S., Broger, C., and Gubernator, K. (1995) Optimierung der biologischen Aktivität von kombinatorischen Verbindungsbibliotheken durch einen genetischen Algorithmus. Angew. Chem. 107, 2452-2454
Wittekind, M., Rajagopal, P., Branchini, B. R., Reizer, J., Saier, M. H. Jr. & Klevit, R. E. (1992) Solution structure of the phosphocarrier protein HPr from *Bacillus subtilis* by two-dimensional NMR spectroscopy*, Protein Sci. 1*, 1363-1376.
Yamamoto, K (1988) Alkaline phosphatase in Electron microscopic cytochemistry and immunochemistry in biomedicine (Ogawa, K., and Barka, T. eds.) CRC Press, Boca Raton.

## Claims

1. Drug assay for the identification of peptides which are specific antimicrobials comprising uncoupling or inhibition of the bacterial phosphotransferase system by either
a) using such peptides as a phosphorylgroup acceptor which is not part of the system and hydrolyses spontaneously or under the influence of non-specific endogenous phosphohydrolases or
b) inhibiting the phosphotransferase system activity with such peptides which block or inactivate at least one of the enzymes of the phosphotransferase system.

2. Drug assay according to claim 1 comprising uncoupling or inhibition of the bacterial phosphotransferase system by using such peptides as a phosphorylgroup acceptor which is not part of the system and hydrolyses spontaneously.

3. Drug assay according to claim 1 or 2 comprising uncoupling of the bacterial phosphotransferase system by using such peptides as a phosphorylgroup acceptor which is not part of the system and hydrolyses under the influence of non-specific endogenous phosphohydrolases.

4. Drug assay according to claim 1 comprising inhibiting the phosphotransferase system activity with said peptides which block or inactivate at least one of the enzymes of the phosphotransferase system.

5. Drug assay according to claim 1 or 4 comprising inhibition of the bacterial phosphotransferase system with said peptides which block at least one of the enzymes of the phosphotransferase system.

6. Drug assay according to claim 1 or 4 comprising inhibition of the bacterial phosphotransferase system with said peptides which inactivate at least one of the enzymes of the phosphotransferase system.

7. Peptide sequences of the formulae:

8. Peptide sequences of the formulae:

9. Pharmaceutical composition comprising one or more of the peptides according to any one of claims 7 and 8.

## Patentansprüche

1. Wirkstoffassay zur Identifizierung von Peptiden, die spezifische antimikrobielle Mittel sind mit einer Entkopplung oder Hemmung des bakteriellen Phosphotransferasesystems, entweder indem a) solche Peptide als Phosphorylgruppenakzeptor, der nicht Teil des Systems ist und spontan oder unter dem Einfluss nicht spezifischer endogener Phosphohydrolasen hydrolysiert, verwendet werden oder b) die Phosphotransferasesystemaktivität durch solche Peptide gehemmt wird, die mindestens eines der Enzyme des Phosphotransferasesystems blockieren oder inaktivieren.

2. Wirkstoffassay nach Anspruch 1, der die Entkopplung oder Hemmung des bakteriellen Phosphotransferasesystems beinhaltet, indem solche Peptide als Phosphorylgruppenakzeptor, der nicht Teil des Systems ist und spontan hydrolysiert, verwendet werden.

3. Wirkstoffassay nach Anspruch 1 oder Anspruch 2, der beinhaltet, dass das bakterielle Phosphotransferasesystem entkoppelt wird, indem solche Peptide als Phosphorylgruppenakzeptor, der nicht Teil des Systems ist und unter dem Einfluss nicht spezifischer endogener Phosphohydrolasen hydrolysiert, verwendet werden.

4. Wirkstoffassay nach Anspruch 1, der beinhaltet, dass die Phosphotransferasesystemaktivität durch diese Peptide gehemmt wird, die mindestens eines der Enzyme des Phosphotransferasesystems blockieren oder inaktivieren.

5. Wirkstoffassay nach Anspruch 1 oder Anspruch 4, der die Hemmung des bakteriellen Phosphotransferasesystems mit diesen Peptiden beinhaltet, die mindestens eines der Enzyme des Phosphotransferasesystems blockieren.

6. Wirkstoffassay nach Anspruch 1 oder Anspruch 4, der die Hemmung des bakteriellen Phosphotransferasesystems mit diesen Peptiden beinhaltet, die mindestens eines der Enzyme des Phosphotransferasesystems inaktivieren.

7. Peptidsequenzen der Formeln

8. Peptidsequenzen der Formeln

9. Pharmazeutische Zusammensetzung enthaltend ein oder mehrere Peptide nach einem der Ansprüche 7 und 8.

## Revendications

1. Essai de médicament pour l'identification de peptides qui sont des antimicrobiens spécifiques comprenant le découplage ou l'inhibition du système phosphotransférase bactérien par
a) utilisation de tels peptides en tant qu'accepteur de groupe phosphoryle qui ne fait pas partie du système et est hydrolysé spontanément ou sous l'influence de phosphohydrolases endogènes non spécifiques ou
b) inhibition de l'activité du système phosphotransférase avec de tels peptides qui bloquent ou inactivent au moins l'une des enzymes du système phosphotransférase.

2. Essai de médicament selon la revendication 1 comprenant le découplage ou l'inhibition du système phosphotransférase bactérien en utilisant des peptides tels qu'un accepteur de groupe phosphoryle qui ne fait pas partie du système et est hydrolysé spontanément.

3. Essai de médicament selon la revendication 1 ou 2 comprenant le découplage ou l'inhibition du système phosphotransférase bactérien en utilisant des peptides tels qu'un accepteur de groupe phosphoryle qui ne fait pas partie du système et est hydrolysé sous l'influence de phosphohydrolases endogènes non spécifiques.

4. Essai de médicament selon la revendication 1 comprenant l'inhibition de l'activité du système phosphotransférase avec lesdits peptides qui bloquent ou inactivent au moins l'une des enzymes du système phosphotransférase.

5. Essai de médicament selon la revendication 1 ou 4 comprenant l'inhibition du système phosphotransférase bactérien avec lesdits peptides qui bloquent au moins l'une des enzymes du système phosphotransférase.

6. Essai de médicament selon la revendication 1 ou 4 comprenant l'inhibition du système phosphotransférase bactérien avec lesdits peptides qui inactivent au moins l'une des enzymes du système phosphotransférase.

7. Séquences peptidiques de formules :

8. Séquences peptidiques de formules :

9. Composition pharmaceutique comprenant un ou plusieurs peptides selon l'une quelconque des revendications 7 et 8.


### SEQUENCE LISTING

(1) GENERAL INFORMATION:
   (i) APPLICANT:
      (A) NAME: ARPIDA
      (B) STREET: Dammstrasse 36
      (C) CITY: Muenchenstein
      (D) STATE: BL
      (E) COUNTRY: Switzerland
      (F) POSTAL CODE (ZIP): CH-4142
      (G) TELEPHONE: +41 61 417 96 60
      (H) TELEFAX: +41 61 417 96 61
   (ii) TITLE OF INVENTION: Target System
   (iii) NUMBER OF SEQUENCES: 32
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ NO: 1:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (xi) SEQUENCE DESCRIPTION: SEQ ID NO: 1:
(2) INFORMATION FOR SEQ ID NO: 2:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (xi) SEQUENCE DESCRIPTION: SEQ ID NO: 2:
   Lys Lys Phe Ala Leu Arg Lys
   1 5
(2) INFORMATION FOR SEQ ID NO: 3:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (xi) SEQUENCE DESCRIPTION: SEQ ID NO: 3:
   Lys Lys Phe Asp Leu Arg Lys
   1 5
(2) INFORMATION FOR SEQ ID NO: 4:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (ix) FEATURE
      (A) NAME/KEY: peptide
      (B) LOCATION: 1
      (C) OTHER INFORMATION: /label= Xaa /note= "wherein Xaa can be any amino acid"
   (ix) FEATURE
      (A) NAME/KEY: peptide
      (B) LOCATION: 2
      (C) OTHER INFORMATION: /label= Xaa /note= "wherein Xaa can be any amino acid"
   (ix) FEATURE
      (A) NAME/KEY: peptide
      (B) LOCATION: 11
      (C) OTHER INFORMATION: /label= Xaa /note= "wherein Xaa can be any amino acid"
   (ix) FEATURE
      (A) NAME/KEY: peptide
      (B) LOCATION: 12
      (C) OTHER INFORMATION: /label= Xaa /note= "wherein Xaa can be any amino acid"
   (xi) SEQUENCE DESCRIPTION: SEQ ID NO: 4:
   Xaa Xaa Lys Lys Trp His Leu Arg Lys Arg Xaa Xaa
   1 5 10
(2) INFORMATION FOR SEQ ID NO: 5:
   (i) SEQUENCE CHARACTERISTICS:
      (A) LENGTH: 8 amino acids
      (B) TYPE: amino acid
      (D) TOPOLOGY: linear
   (ii) MOLECULE TYPE: peptide
   (xi) SEQUENCE DESCRIPTION: SEQ ID NO: 5:
   Lys Lys Trp His Leu Arg Lys Arg
   1 5
(2) INFORMATION FOR SEQ ID NO: 6:
   (i) SEQUENCE CHARACTERISTICS:
      (A) LENGTH: 8 amino acids
      (B) TYPE: amino acid
      (D) TOPOLOGY: linear
   (ii) MOLECULE TYPE: peptide
   (xi) SEQUENCE DESCRIPTION: SEQ ID NO: 6:
   Lys Gly Trp His Lys Arg Lys Lys
   1 5
(2) INFORMATION FOR SEQ ID NO: 7:
   (i) SEQUENCE CHARACTERISTICS:
      (A) LENGTH: 8 amino acids
      (B) TYPE: amino acid
      (D) TOPOLOGY: linear
   (ii) MOLECULE TYPE: peptide
   (xi) SEQUENCE DESCRIPTION: SEQ ID NO: 7:
   Lys Lys Trp His Arg Arg Lys Lys
   1 5
(2) INFORMATION FOR SEQ ID NO: 8:
   (i) SEQUENCE CHARACTERISTICS:
      (A) LENGTH: 8 amino acids
      (B) TYPE: amino acid
      (D) TOPOLOGY: linear
   (ii) MOLECULE TYPE: peptide
   (xi) SEQUENCE DESCRIPTION: SEQ ID NO: 8:
   Lys Lys Trp His Lys Arg Lys Lys
   1 5
(2) INFORMATION FOR SEQ ID NO: 9:
   (i) SEQUENCE CHARACTERISTICS:
      (A) LENGTH: 8 amino acids
      (B) TYPE: amino acid
      (D) TOPOLOGY: linear
   (ii) MOLECULE TYPE: peptide
   (xi) SEQUENCE DESCRIPTION: SEQ ID NO: 9:
   Lys Lys Phe His Ile Arg Lys Arg
   1 5
(2) INFORMATION FOR SEQ ID NO: 10:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ NO: 10:
   Pro Asn Gly Leu His Thr Arg Pro Ala
   1 5
(2) INFORMATION FOR SEQ ID NO: 11:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (ix) FEATURE
      (A) NAME/KEY: peptide
      (B) LOCATION: 1
      (C) OTHER INFORMATION: /label= Pro /note= "wherein a biotin molecule is covalently linked to Pro at position 1"
   (xi) SEQUENCE DESCRIPTION: SEQ ID NO: 11:
   Pro Asn Gly Leu His Thr Arg Pro Ala
   1 5
(2) INFORMATION FOR SEQ ID NO: 12:
   (i) SEQUENCE CHARACTERISTICS:
      (A) LENGTH: 4 amino acids
      (B) TYPE: amino acid
      (D) TOPOLOGY: linear
   (ii) MOLECULE TYPE: peptide
   (xi) SEQUENCE DESCRIPTION: SEQ ID NO: 12
   Trp His Lys Arg
   1
(2) INFORMATION FOR SEQ ID NO: 13
   (i) SEQUENCE CHARACTERISTICS:
      (A) LENGTH: 4 amino acids
      (B) TYPE: amino acid
      (D) TOPOLOGY: linear
   (ii) MOLECULE TYPE: peptide
   (ix) FEATURE
      (A) NAME/KEY: peptide
      (B) LOCATION: 1
      (C) OTHER INFORMATION: /label= Trp /note= "wherein Trp is acetylated"
   (xi) SEQUENCE DESCRIPTION: SEQ ID NO: 13:
   Trp His Lys Arg
   1
(2) INFORMATION FOR SEQ NO: 14:
   (i) SEQUENCE CHARACTERISTICS:
      (A) LENGTH: 15 amino acids
      (B) TYPE: amino acid
      (D) TOPOLOGY: linear
   (ii) MOLECULE TYPE: peptide
   (xi) SEQUENCE DESCRIPTION: SEQ ID NO: 14:
(2) INFORMATION FOR SEQ ID NO: 15:
   (i) SEQUENCE CHARACTERISTICS:
      (A) LENGTH: 15 amino acids
      (B) TYPE: amino acid
      (D) TOPOLOGY: linear
   (ii) MOLECULE TYPE: peptide
   (ix) FEATURE
      (A) NAME/KEY: peptide
      (B) LOCATION: 11
      (C) OTHER INFORMATION: /label= Trp /note= "wherein Trp is acetylated"
   (xi) SEQUENCE DESCRIPTION: SEQ ID NO: 15:
   Ser Gly Arg Lys Ser Thr Arg Val His His Trp Leu Leu Val Leu
   1 5 10 15
(2) INFORMATION FOR SEQ ID NO: 16:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO: 16:
   Lys Met Ser Arg His Arg Lys Pro Gly Ala
   1 5 10
(2) INFORMATION FOR SEQ ID NO: 17:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ NO: 17:
   Ser Ser Leu Arg Gly His Arg Trp Val Tyr
   1 5 10
(2) INFORMATION FOR SEQ ID NO: 18:
   (i) SEQUENCE CHARACTERISTICS:
      (A) LENGTH: 10 amino acids
      (B) TYPE: amino acid
      (D) TOPOLOGY: linear
   (xi) SEQUENCE DESCRIPTION: SEQ ID NO: 18:
   Lys Ile Ser Arg His Gly Lys Arg Gly Lys
   1 5 10
(2) INFORMATION FOR SEQ ID NO: 19:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ NO: 19:
   Lys Ile Ser Arg His Gly Arg Pro Thr Gly
   1 5 10
(2) INFORMATION FOR SEQ ID NO: 20:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO: 20:
   Arg Ile His Phe Ile Pro Arg Arg Gly Arg
   1 5 10
(2) INFORMATION FOR SEQ ID NO: 21:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (xi) SEQUENCE DESCRIPTION: SEQ ID NO: 21:
   Arg Leu His Tyr Leu Phe
   1 5
(2) INFORMATION FOR SEQ ID NO: 22:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO: 22:
   Asp Gly Ala Arg Leu His Tyr Leu Phe
   1 5
(2) INFORMATION FOR SEQ ID NO: 23:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (ix) FEATURE
      (A) NAME/KEY: peptide
      (B) LOCATION: 1
      (C) OTHER INFORMATION: /label= Xaa /note= "wherein Asp is acetylated"
   (xi) SEQUENCE DESCRIPTION: SEQ ID NO: 23:
   Asp Gly Ala Arg Leu His Tyr Leu Phe
   1 5
(2) INFORMATION FOR SEQ ID NO: 24:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (xi) SEQUENCE DESCRIPTION: SEQ ID NO: 24:
   Arg His Trp Ser Ile Phe
   1 5
(2) INFORMATION FOR SEQ ID NO: 25:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (xi) SEQUENCE DESCRIPTION: SEQ ID NO: 25:
   Arg His Arg Thr Leu Phe
   1 5
(2) INFORMATION FOR SEQ ID NO: 26:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (xi) SEQUENCE DESCRIPTION: SEQ ID NO: 26:
   Arg His Tyr Leu Leu Phe
   1 5
(2) INFORMATION FOR SEQ ID NO: 27:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (xi) SEQUENCE DESCRIPTION: SEQ ID NO: 27:
   Arg His Ile Thr Ser Leu
   1 5
(2) INFORMATION FOR SEQ NO: 28:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (xi) SEQUENCE DESCRIPTION: SEQ ID NO: 28:
(2) INFORMATION FOR SEQ ID NO: 29:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (xi) SEQUENCE DESCRIPTION: SEQ NO: 29:
   Met Ser Arg His Arg Asn
   1 5
(2) INFORMATION FOR SEQ ID NO: 30:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO: 30:
   Pro Asn Gly Leu His Thr Arg Pro Ala
   1 5
(2) INFORMATION FOR SEQ ID NO: 31:
   (i) SEQUENCE CHARACTERISTICS:
      (A) LENGTH: 13 amino acids
      (B) TYPE: amino acid
      (D) TOPOLOGY: linear
   (ii) MOLECULE TYPE: peptide
   (xi) SEQUENCE DESCRIPTION: SEQ ID NO: 31:
   Met Arg Leu Leu Lys Thr Leu Cys Phe Gly Leu Cys Gly
   1 5 10
(2) INFORMATION FOR SEQ ID NO: 32:
   (i) SEQUENCE CHARACTERISTICS:
      (A) LENGTH: 14 amino acids
      (B) TYPE: amino acid
      (D) TOPOLOGY: linear
   (ii) MOLECULE TYPE: peptide
   (ix) FEATURE
      (A) NAME/KEY: peptide
      (B) LOCATION: 1
      (C) OTHER INFORMATION: /label= Met
         /note= "wherein Met is acetylated"
   (xi) SEQUENCE DESCRIPTION: SEQ ID NO: 32:
   Met Arg Leu Leu Lys Thr Leu Cys Phe Val Gly Leu Cys Gly
   1 5 10